Europäisches Patentamt

European Patent Office

Office européen des brevets

(11) Numéro de publication : **0 517 600 B1**

(19)

## (12) FASCICULE DE BREVET EUROPEEN

(45) Date de publication du fascicule du brevet :
**01.12.93 Bulletin 93/48**

(51) Int. Cl.$^5$ : **G01T 1/166, A61B 6/00**

(21) Numéro de dépôt : **92401544.9**

(22) Date de dépôt : **04.06.92**

(54) Procédé d'acquisition tomographique, à deux détecteurs, à centre de visée distinct du centre de rotation.

(30) Priorité : **07.06.91 FR 9106962**

(43) Date de publication de la demande :
**09.12.92 Bulletin 92/50**

(45) Mention de la délivrance du brevet :
**01.12.93 Bulletin 93/48**

(84) Etats contractants désignés :
**AT DE ES GB IT NL**

(56) Documents cités :
**EP-A- 0 266 846**
**US-A- 4 652 759**
**US-A- 4 692 625**

(73) Titulaire : **SOPHA MEDICAL**
**9, Place de la Madeleine**
**F-75008 Paris (FR)**

(72) Inventeur : **Pierfitte, Michel, CABINET**
**BALLOT-SCHMIT**
**7, rue Le Sueur**
**F-75116 Paris (FR)**
Inventeur : **Delorme, Pierre, CABINET**
**BALLOT-SCHMIT**
**7, rue Le Sueur**
**F-75116 Paris (FR)**

(74) Mandataire : **Schmit, Christian Norbert Marie**
**et al**
**Cabinet Ballot-Schmit 7, rue Le Sueur**
**F-75116 Paris (FR)**

EP 0 517 600 B1

Jouve, 18, rue Saint-Denis, 75001 PARIS

## Description

La présente invention a pour objet un procédé d'acquisition d'images avec une gamma caméra au cours d'un examen tomographique en médecine nucléaire.

L'invention est particulièrement intéressante si l'auto-atténuation des rayons gamma dans le patient est importante: en particulier pour les tomographies du myocarde, donc pour des organes localisés sur le côté du corps humain. Cependant, ceci ne peut en aucun cas constituer une limitation de l'invention à ce type d'utilisation.

Des gamma caméras sont par exemple décrites dans le brevet américain de ANGER n° 3 011 057. Une gamma caméra est un appareil comportant une embase tournante, fixe ou mobile par rapport au sol, et qui porte, au bout d'un bras, un détecteur, dit encore tête détectrice. Ce détecteur et muni d'un réseau de tubes photo-multiplicateurs dont les faces d'entrée, juxtaposées les unes aux autres, constituent la surface de détection de la tête détectrice et son champ de détection.

Le principe de l'examen est le suivant. On injecte un produit radioactif dans un patient à examiner. Ce produit est par exemple du Thallium pour l'examen du myocarde. L'émission radioactive vient exciter un cristal scintillateur du détecteur qui convertit l'énergie des photons gamma en une énergie lumineuse détectable par les tubes photo-multiplicateurs. Le cristal scintillateur est précédé, d'une manière classique, d'un collimateur définissant une direction de visée et caractérisé par un foyer. Ce foyer est rejeté à l'infini dans le cas des collimateurs à trous parallèles, droits ou inclinés. Le foyer est à une distance finie, positive ou négative, dans le cas des collimateurs convergents ou divergents. Le foyer peut être décentré par rapport à une direction centrale de visée.

Les scintillations émises sont détectées par les tubes photo-multiplicateurs qui produisent des signaux électriques dépendant de l'intensité lumineuse reçue. En effectuant sur l'ensemble de ces signaux électriques des localisations barycentriques, on peut, d'une manière connue, déterminer la localisation X Y de l'origine de la scintillation dans le champ de détection. On réalise alors une acquisition incrémentale en cumulant le nombre de scintillations (ou coups) détectés par élément de localisation dit pixel.

En laissant la tête détectrice dans une position donnée pendant un certain temps au-dessus du corps examiné, on peut alors, pour un angle de vue donné, dit projection, obtenir une image révélatrice de la concentration du produit émetteur dans le corps. L'examen tomographique consiste à acquérir une image par angle de vue, pour un grand nombre d'angles de vue, régulièrement espacés sur un secteur angulaire d'au moins 180°. On sait ensuite, avec des algorithmes de calcul, notamment la rétro-projection filtrée, reconstituer l'image du volume examiné.

Pour les applications cardiaques, compte tenu du mouvement du coeur, on procède par ailleurs à une synchronisation des acquisitions. Pour augmenter la sensibilité de la caméra, on a pris l'habitude d'utiliser une embase tournante munie de deux têtes détectrices au lieu d'une seule. Ces deux têtes sont en vis à vis l'une de l'autre et tournent, ensemble, autour du patient examiné. Elles contribuent toutes les deux à l'acquisition des projections. Les directions de visée des deux détecteurs coïncident alors. Elles passent par l'axe de rotation du système.

Ce type de géométrie n'améliore pas la sensibilité pour les examen cardiaques pour les raisons suivantes. L'atténuation est d'autant plus forte que l'énergie de l'isotope utilisé est faible. En conséquence, les projections du secteur angulaire de 180° acquis (qui donne les projections les plus près de l'organe à étudier, et qui minimise l'auto-atténuation des tissus interposés entre cet organe et le détecteur) sont beaucoup plus significatives que les projections du secteur angulaire opposé. En outre l'éventuelle utilisation du secteur opposé lors de la retro-projection filtrée dégrade le résultat obtenu. Il apparaît ainsi que le deuxième détecteur est inutile dans ce cas.

Cet inconvénient se retrouve par ailleurs avec les gamma caméras à trois ou quatre têtes, pour lesquelles il y a toujours une ou deux têtes inutiles.

L'invention a pour objet de remédier aux inconvénients cités, en proposant, avec une caméra à deux têtes une géométrie de caméra et une cinématique d'examen différentes de manière à doubler la sensibilité. Le principe de l'invention consiste à décaler le centre de visée, défini comme l'intersection des deux directions de visée, par rapport à l'axe de rotation de la gamma caméra. Ceci peut être obtenu par exemple de deux façons. D'une manière préférée, les champs de détection des deux têtes détectrices ne sont plus parallèles. Cette géométrie est obtenue par des angulations symétriques (de préférence) des têtes détectrices par rapport au plan horizontal passant par l'axe de rotation de l'embase alors que cette embase est dans une orientation verticale. D'une autre manière, des collimateurs à trous inclinés permettent d'obtenir le décalage du centre de visée par rapport à l'axe de rotation, alors que les champs de détection peuvent rester parallèles. Dans ce cas il faut autant de collimateurs que de valeur de décalage possible. Bien sûr on peut aussi associer les deux techniques et obtenir l'écart choisi en angulant d'une part les têtes et en munissant les détecteurs de collimateurs à trous inclinés.

On montre qu'alors on peut diviser par deux l'angle de rotation autour du corps. En particulier dans une variante préférée de la méthode, lorsque l'angulation des détecteurs ou la direction de visée des collimateurs sont égales à 45°, l'acquisition tomographique de 180° est obtenue par une rotation de seulement 90° de l'embase. Le support patient est alors animé d'un mouvement ascensionnel tandis que l'embase est animée d'un mouvement de translation latérale (ou vice versa), en synchronisme avec la rotation des détecteurs. Le mouvement relatif du patient par rapport à l'embase ou de l'embase par rapport au patient étant un arc de cercle.

L'invention a donc pour objet un procédé d'acquisition tomographique d'images, au cours d'un examen de médecine nucléaire mené avec une gamma caméra à deux têtes détectrices, ces deux têtes étant maintenues en vis à vis l'une de l'autre par une embase tournant autour d'un axe de rotation, caractérisé en ce que

- on munit chacune des deux têtes d'un axe d'angulation de sa direction de visée, ces deux axes d'angulation étant parallèles à l'axe de rotation de l'embase, cet axe de rotation de l'embase étant contenu dans le plan défini par ces deux axes d'angulation,
- on oriente en angulation la direction de visée de chacune des têtes, définissant ainsi un centre de visée présentant un écart par rapport à cet axe de rotation.

L'invention sera mieux comprise à la lecture de la description qui suit et à l'examen des figures qui l'accompagnent. Celles-ci ne sont données qu'à titre indicatif et nullement limitatif de l'invention.

Les figures montrent :
- Figure 1: une gamma caméra à deux têtes utilisable pour mettre en oeuvre le procédé de l'invention ;
- Figures 2a et 2b: des représentations schématiques comparées des procédés d'acquisitions de l'état de la technique et de l'invention respectivement ;
- Figures 3a à 3c et 4a à 4c: des représentations de différentes positions de l'ensemble des têtes détectrices dans une variante préférée de mise en oeuvre du procédé inventé;
- Figures 5 et 6: des diagrammes géométriques permettant d'expliquer la cinématique de déplacement des têtes au cours d'une tomographie;
- Figures 7a et 7b: des diagrammes géométriques montrant une variante de mise en oeuvre du procédé de l'invention.

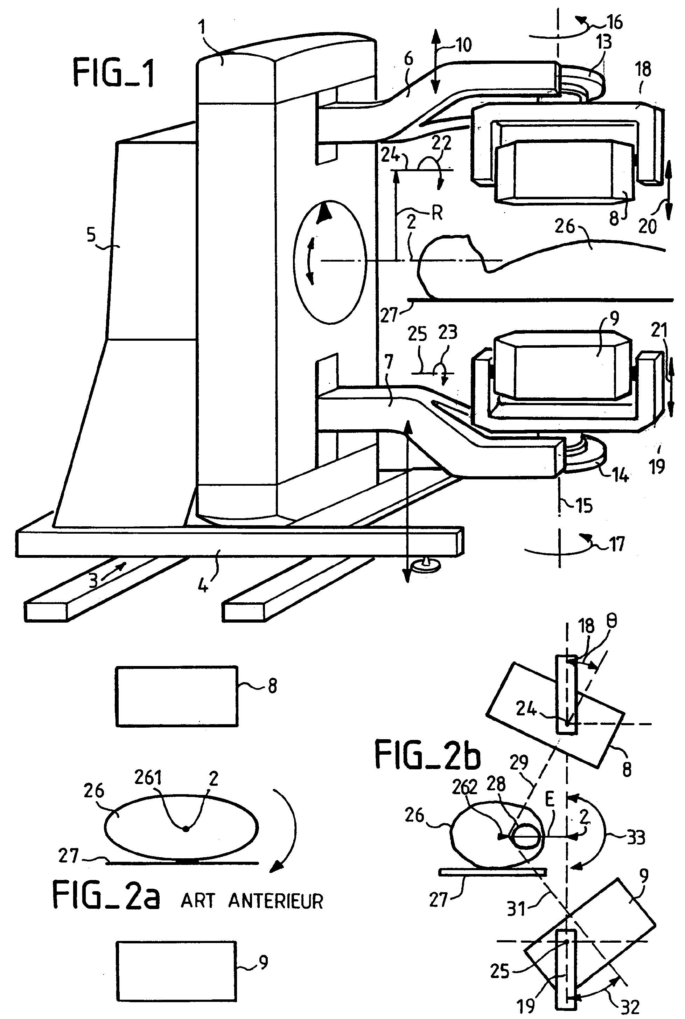

La figure 1 montre une gamma caméra utilisable pour la mise en oeuvre du procédé de l'invention. Cette gamma caméra comporte une embase tournante 1 susceptible de tourner autour d'un axe de rotation 2, du fait de son maintien en rotation sur un bâti 3 muni d'un socle 4 et d'un piédestal 5. Ce mouvement sera appelé rotation dans le reste de la description. L'embase 1 comporte deux bras porteurs respectivement 6 et 7 portant chacun une tête détectrice respectivement 8 et 9 de la gamma caméra. D'une manière connue, ces bras peuvent s'écarter ou se rapprocher l'un de l'autre, d'une manière symétrique, selon le sens de la double flèche 10 par un mécanisme de mouvement radial contenu à l'intérieur de l'embase 1 et motorisé par ailleurs par des moteurs électriques.

Chaque bras 6 et 7 possède en extrémité une couronne respectivement 13 et 14. A chacune des couronnes 13 et 14 est fixé respectivement un étrier 18 et 19. Les deux flancs de chacun de ces étriers comportent un mécanisme susceptible d'une part d'autoriser un mouvement télescopique, selon le sens des flèches 20 et 21, des têtes détectrices 8 et 9, ainsi qu'une rotation, appelée par la suite angulation, respectivement 22 et 23 autour d'axes 24 et 25 des têtes détectrices. Ces mouvements sont par ailleurs décrits dans une autre demande de brevet déposée le même jour par le même demandeur. Les axes d'angulation passent à l'aplomb du milieu du champ de détection de chacune des têtes. Ils sont parallèles à ces champs. Le mouvement télescopique permet de déplacer de manière indépendante (et non plus symétriquement comme pour le mouvement radial 10 des bras 6 et 7) chacune des têtes 8 et 9. Quand les axes d'angulation sont à l'extrémité des étriers 18 et 19, les têtes détectrices peuvent subir un mouvement d'angulation 22 ou 23 de + 90° ou - 90°. Dans le procédé de l'invention on verra qu'on choisit de préférence une angulation comprise entre 30° et 45° pour une acquisition tomographique avec les meilleures données possibles, mais pouvant aller de 5° à 75° dans des modes dégradés. Il reste alors de la marge pour effectuer un mouvement télescopique.

Les têtes détectrices ont dans un exemple un champ de détection rectangulaire. La grande longueur de ce rectangle est parallèle à l'axe d'angulation 24.

Les mouvements télescopique et de rotation sont de préférence motorisés par des moteurs électriques. Les mouvement d'angulation sont manuels avec possibilité de sélection parmi des positions prédéfinies. Dans un exemple ces position prédéfinies sont constituées par des encoches réalisées en périphérie de plaques circulaires concentriques à l'axe d'angulation et solidaires de chacune des têtes. Deux crans peuvent venir s'engager dans ces encoches et ainsi maintenir dans des positions prédéfinies l'angulation des têtes.

Un patient examiné 26 est placé, sensiblement entre les deux têtes 8 et 9, sur un lit porte-patient 27.

La figure 2a montre schématiquement le principe de réalisation d'une tomographie dans l'art antérieur. Les deux têtes 8 et 9 sont normalement placées en vis-à-vis l'une de l'autre, de part et d'autre du patient examiné 26. L'axe de rotation 2 de l'embase est confondu sensiblement avec le centre de visée 261. Par contre, dans l'invention figure 2b, l'axe de rotation 2 est décalé d'un écart E par rapport au centre de visée 262. En particulier pour un patient avec un coeur 28 à gauche (ici on le voit par les pieds) l'axe de rotation de l'embase est décalé sur la gauche de ce patient. On observe encore sur la figure 2b les traces des axes 24 et 25 alignées avec la trace de l'axe de rotation 2. Ces trois axes sont dans un même plan. On constate que la tête détectrice 8 a été tournée en angulation et que sa direction principale de visé 29 passe par le centre de visée 262. L'angulation Θ a une valeur d'environ 15° à 45°. On retrouve les mêmes éléments pour la tête détectrice 9 avec une direction de visé 31 et une angulation 32. La rotation de l'embase est effectuée, selon la flèche 33, autour du centre de rotation 2.

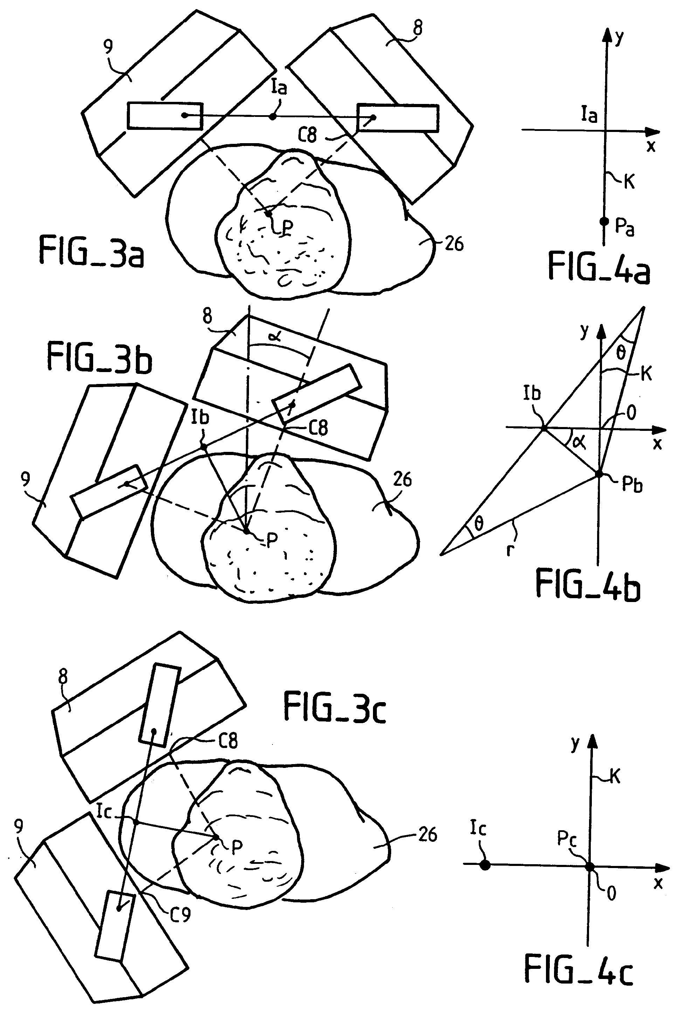

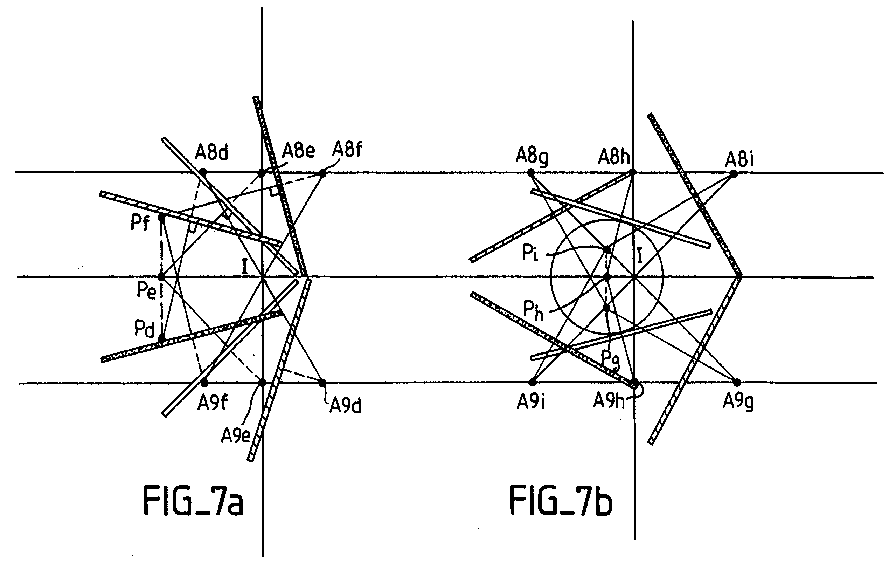

On va expliquer maintenant à l'aide des figures 3a à 3c, 4a à 4c, 5 et 6, comment, dans un mode préféré, on met en oeuvre l'invention. A l'aide de la figure 7 on montrera une variante. Quand on décrira des angulations il faudra aussi comprendre l'utilisation alternative de collimateurs à trous inclinés, ou même un mélange des deux. Dans ces cas, l'axe d'angulation s'entend comme la droite parallèle à l'axe de rotation et passant par le centre du champ du détecteur.

Les champs de détection sont de dimension inférieure ou égale au détecteur.

Le principe du mode préféré, est de conserver inchangé le dessin géométrique constitué par le centre de visée P, la projection I de l'axe de rotation 2 dans le plan de rotation tomographique, et les centres C8 et C9 des champs de détection des détecteurs 8 et 9, alors que l'appareil tourne autour du patient 26. On obtient alors une tomographie à centre de visée constant P. On peut en attendre les résultats les plus rigoureux au moment de la reconstruction des images.

Les figures 3 et 4, indicées a b et c, montrent trois positions initiales, intermédiaires et finales de l'appareil au cours de sa rotation d'exploration dans le mode préféré. Pour arriver à ce résultat on déplace le lit verticalement. Ainsi, figures 4a à 4c, la position du point P est passée d'une position basse Pa à une position haute Pc mesurée par rapport à un repère fixe K de centre O. Elle est passée par une position intermédiaire Pb. Par contre l'axe de rotation de l'embase s'est déplacé latéralement progressivement vers la gauche, de sa position initiale Ia, vers sa position finale Ic. Ces deux mouvements sont connus en eux mêmes, le lit étant muni d'un dispositif d'ascension et l'embase étant susceptible de se déplacer latéralement en faisant avec le bâti 4 sur des rails. Le mouvement ascensionnel du lit se fait suivant l'axe Y du repère K. Le déplacement latéral du bâti se fait suivant l'axe X du repère K.

Pour pouvoir pratiquer une tomographie du coeur on place l'appareil pour que le centre de visée P soit sensiblement au centre du patient. On explore avec une tête un secteur angulaire au cours de la rotation de l'embase. L'exploration de ce secteur est complétée par celle effectuée par la deuxième tête. Pour que la reconstruction des images soit correcte il faut que les deux explorations soient contiguës ou se recouvrent. Il faut donc que chaque tête explore au moins un secteur angulaire de 90°.

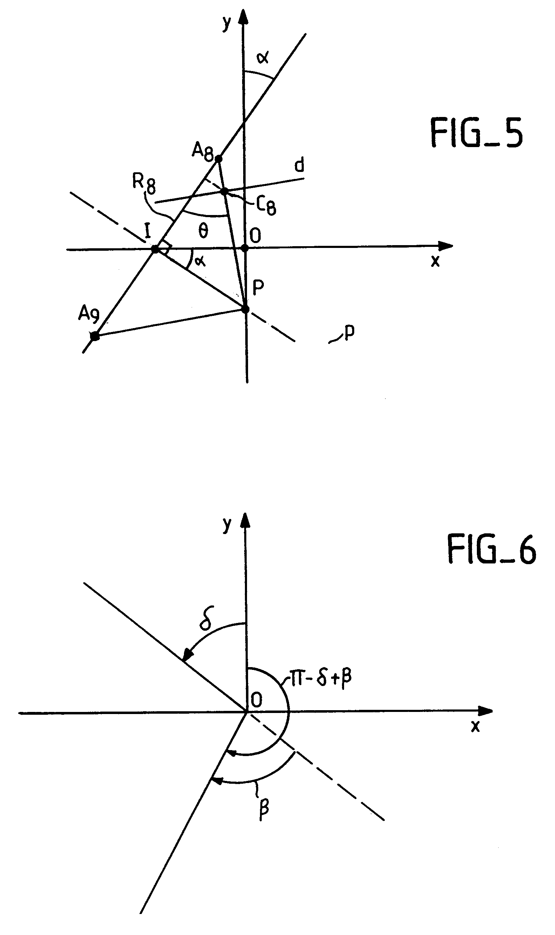

La figure 5 montre le dessin géométrique évoqué avec en plus l'angle Θ d'angulation d'une tête détectrice, dont la dimension du détecteur est d, la longueur du champ de détection étant inférieure. L'angle α de rotation de l'embase est l'angle que fait la droite A8IA9 avec l'axe Y, A8 et A9 étant les traces des axes d'angulation dans le plan tomographique. La figure 5 permet de calculer les contraintes d'encombrement et de faisabilité des examen avec le procédé de l'invention. Pour simplifier ces calculs on a choisi de placer le centre de visée P sur une perpendiculaire p à la droite passant par A8IA9. Mais il pourrait en être autrement notamment si les angulations des deux têtes ne sont pas égales. On appelle r la distance du centre de visée P au centre du champ C de la tête détectrice. On appelle R le rayon imposé par l'embase pour écarter symétriquement les têtes détectrices. On appelle $R_8$ (ou $R_9$) le rayon propre à la tête 8 (ou 9). Sa valeur est égale à IA8 - A8C8. La position du point A8 dépend de la valeur du mouvement télescopique de détecteur 8. Dans un exemple d vaut 45,2 cm, AC vaut 13 cm et R est compris entre 8 et 35 cm.

Pour éviter que les têtes n'entrent en collision il faut déjà que r soit plus grand que d/2 . tgΘ

La figure 6 montre les conditions pratiques de l'acquisition des vues. Pour des raisons cliniques le début de l'exploration est décalé d'un angle δ par rapport à la verticale. L'angle total d'exploration vaut au moins 180° (pour satisfaire aux contraintes des calculs de reconstruction). Par ailleurs on peut vouloir dépasser la valeur de 180° de la valeur d'un angle β. En pratique δ vaut 45° et β peut valoir de l'ordre de 60°. On peut anguler chacune des deux têtes d'un angle respectivement $\Theta_1$ et $\Theta_2$ différent. Dans ce cas on choisit ces deux angles tels que leur somme soit égale à $(\pi - \beta)/2$.

La procédure d'examen est très simple. Le patient se couche sur le lit. Les deux têtes sont placées au dessus de lui (sensiblement symétriquement de part et d'autre de la verticale). Et l'embase explore 90° au moins en tournant du coté où se trouve l'organe à examiner. Pour que les secteurs angulaires d'exploration soient contigüs, on a calculé que β doit être plus grand que π - 4.Θ. Comme par ailleurs au delà de β=60° une auto-atténuation importante ne permet plus d'enregistrer des données utiles, on déduit en utilisant la relation précédente que Θ doit être supérieur à 30° pour avoir de bons résultats. En effet, les informations détectées par la tête détectrice inférieure ne sont plus significatives (les émissions radioactives sont trop atténuées vers la droite par la présence de la colonne vertébrale). On trouve alors les valeurs suivantes pour les angles d'angulation Θ, de complément d'exploration β, et de rotation S de l'embase

| Θ | β | S |
|---|---|---|
| 45° | 0° | 90° |
| 30° | 60° | 120° |

Dans ces deux cas l'examen est plus rapide parce que la rotation est inférieure à 180°.

On peut écrire, voir figure 5, que le décalage latéral OI de l'embase tournante vaut OI = IP cos $\alpha$. De même on peut écrire que l'altitude du lit est OP = IP sin$\alpha$. Par ailleurs on sait que IP = IA8 tg Θ. Ceci permet d'écrire simplement toutes les relations qui lient les déplacements des pièces mobiles de la gamma caméra pendant la mise en oeuvre du procédé de l'invention. En effet l'écart IP est la donnée fondamentale du procédé et reste constant pendant l'examen. IP varie d'un examen à l'autre en fonction de la morphologie du patient. On peut déterminer la valeur de cet écart IP de préférence de la manière suivante. On fixe d'abord Θ à une valeur choisie, les deux têtes étant écartées l'une de l'autre. On place ensuite la gamma caméra dans la position initiale de l'examen à pratiquer. Par exemple cette position est celle de la figure 3a. On rapproche alors les têtes jusqu'à ce qu'on vienne presque toucher le patient. On peut alors mesurer ou connaître par construction le rayon puisqu'on connaît IaC8. Cette connaissance peut être obtenue par un capteur mesurant l'amplitude du mouvement radial. De la connaissance du rayon on déduit par construction les valeurs de IA8 et IP. On en déduit ensuite la course utile du lit en hauteur et de l'embase en déplacement latéral. On déduit aussi de ces dernières relations les contraintes qui lient les positions relatives en élévation du lit et en translation latérale de l'embase tournante à l'angle $\alpha$ de rotation de cette embase tournante. Ainsi dans le mode préféré, Θ vaut 45° (tgΘ=1), IP = IA8, l'angle $\alpha$ de départ vaut -90°, l'angle $\alpha$ final vaut 0°.

De préférence les motorisations de ces déplacements en élévation et en translation sont asservies avec avec des asservissements dont les fonctions de transfert sont celles indiquées ci-dessus, en prenant $\alpha$ comme variable et IP comme constante.

Les figures 7a et 7b montrent des variantes de mise en oeuvre du procédé de l'invention dans lesquelles l'embase n'est pas déplacée latéralement. Seul le lit est monté d'une position basse à une position haute au cours de l'examen, pendant que l'embase tourne sur elle-même. On gagne alors un mouvement qui n'est pas à exécuter. Dans l'exemple de la figure 7a l'angulation de chacune des têtes est de 45°. Alors que le centre de rotation de l'embase reste immobile au point I, le centre de visée (correspondant avec le centre du corps du patient) s'élève verticalement de la position Pd à la position Pe puis Pf. Pendant ce mouvement, les projections dans le plan de tomographie des axes d'angulation des têtes 8 et 9 occupent successivement et respectivement les positions A8d à A8f et A9d à A9f. Ces positions sont alignées sur deux droites parallèles et horizontales. Pendant ce mouvement, on joue sur le rayon pour premièrement resserrer symétriquement les têtes l'une de l'autre (de la position d à la position e), puis on les écarte (de la position e à la position f). Ceci est visible sur la figure 7a où, dans les positions d et f les extrémités les plus proches l'une de l'autre des détecteurs sont éloignés l'une de l'autre, alors qu'elles viennent presque au contact dans la position e.

Ceci permet par ailleurs de régler les têtes. Ainsi, on angule d'abord les têtes de 45°. Puis on les rapproche jusqu'à ce qu'elles se touchent presque, en jouant sur le rayon, et alors que l'embase est verticale. On connaît alors l'altitude des droites D8 et D9 sur lesquelles doivent se déplacer les axes d'angulation. On déduit l'équation du déplacement en hauteur du lit et l'équation de la variation du rayon en fonction de $\alpha$ par un calcul trigonométrique. En effet, dans cette solution, le produit IA.cos$\alpha$ est une valeur constante. Le rayon est donc proportionnel à 1/cos$\alpha$.

Sur la figure 7b on a cherché à réduire le débattement en hauteur du lit au cours de l'exploration, toujours en maintenant le centre de rotation I immobile. On a choisi alors un angle d'angulation faible: 15°. Dans ce cas le déplacement en hauteur du lit est faible mais la variation du rayon est plus sensible. On reconnaît aussi sur cette figure le centre de rotation I placé au milieu des segments A8-A9, les segments A8-P et A9-P étant perpendiculaires aux champs de détection des têtes.

Pour compléter ces deux solutions générales (embase en mouvement de translation et lit en mouvement d'ascension, ou lit seul en mouvement de d'ascension), on indique qu'il est possible par ailleurs de provoquer un déplacement relatif de l'axe de rotation I de l'embase dans le plan tomographique suivant un cercle de centre P et de ryon IP.

Enfin on peut effectuer une autre exploration totale dans laquelle, au cours de la rotation, ni le lit ni l'axe de rotation de l'embase ne sont déplacés. Avec une telle acquisition le centre de visée physique n'est pas fixe dans le corps du patient. On peut montrer néanmoins que dans le champ de détection on peut identifier une surface de détection réduite, glissante dans ce champ, qui permette de définir un centre de visée "utile" toujours fixe. Il y a alors conformément à l'invention toujours un écart constant entre ce centre de visée utile et l'axe de rotation. On obtient un centre de visée utile constant en extrayant un champ utile du champ de détection en fonction de l'angle de rotation $\alpha$.

**Revendications**

**1.** Procédé d'acquisition d'images(36) tomographiques, au cours d'un examen de médecine nucléaire mené avec une gamma caméra à deux têtes (8, 9) détectrices, ces deux têtes étant maintenues (1, 6, 7) en vis à vis l'une de l'autre par une embase tournant autour d'un axe de rotation, caractérisé en ce que

- on munit chacune des deux têtes d'un axe d'angulation de sa direction de visée, ces deux axes d'angulation étant parallèles à l'axe de rotation de l'embase, cet axe de rotation de l'embase étant contenu dans le plan défini par ces deux axes d'angulation,
- on oriente (24, 25) en angulation la direction de visée de chacune des têtes, définissant ainsi un centre de visée (262) présentant un écart (E) par rapport à cet axe de rotation.

2. Procédé selon la revendication 1, caractérisé en ce que l'examen est une tomographie, en ce qu'on explore le corps en faisant tourner l'embase autour d'un axe de rotation, et en ce que le centre de visée se trouve hors de l'axe de rotation, dans un plan transverse à cet axe.

3. Procédé selon la revendication 1 ou la revendication 2, caractérisé en ce que

- on fait tourner ces deux têtes ensemble d'un angle nettement inférieur à 180°.

4. Procédé selon l'une quelconque des revendications 1 à 3, caractérisé en ce que

- on déplace le centre de rotation et le centre de visée au cours de la rotation, et en ce que
- on maintient la valeur de l'écart au cours de la rotation.

5. Procédé selon la revendication 4, caractérisé en ce qu'on déplace le centre de rotation horizontalement pendant qu'on déplace le centre de visée verticalement.

6. Procédé selon l'une quelconque des revendications 1 à 5, caractérisé en ce que

- on oriente les directions de visée des deux têtes de telle façon que leurs directions principales de visée fassent entre elles un angle de 90°.

7. Procédé selon l'une quelconque des revendications 1 à 6, caractérisé en ce que

- on désoriente les directions de visée de deux têtes avec un angle égal par rapport à des orientations de base qu'elles ont lorsque ces directions de visée sont parallèles.

8. Procédé selon l'une quelconque des revendications 1 à 7, caractérisé en ce que

- on angule les deux têtes avec un angle égal par rapport à des orientations de base qu'elles ont lorsqu'elles sont parallèles.

9. Procédé selon l'une quelconque des revendications 1 à 8, caractérisé en ce que

- on angule chacune des deux têtes d'un angle respectivement $\Theta_1$ et $\Theta_2$ tel que la somme de ces deux angles soit égale à $(\pi - \beta)/2$, où $\beta$ est un secteur complémentaire d'exploration au delà d'un secteur principal de 180°.

10. Procédé selon l'une quelconque des revendications 1 à 9, caractérisé en ce que

- on désoriente les directions de visées avec des angles prédéfinis.

11. Procédé selon l'une quelconque des revendications 1 à 10, caractérisé en ce que

- on désoriente les directions de visée à la fois en angulant les têtes détectrice et en munissant leur surface de détection d'un collimateur incliné.

12. Procédé selon l'une quelconque des revendications 1 à 11, caractérisé en ce que

- on commence l'examen en plaçant le lit sensiblement symétriquement sous les deux têtes détectrices.

13. Procédé selon l'une quelconque des revendications 1 à 12, caractérisé en ce que

- on élève le patient sans déplacer latéralement l'embase.

14. Procédé selon l'une quelconque des revendications 1 à 12, caractérisé en ce que

- on déplace l'embase en un mouvement circulaire autour du patient sans déplacer le lit.


**Patentansprüche**

1. Tomographisches Aufnahmeverfahren für eine nuklearmedizinische Untersuchung, das mit einer Gammakamera mit zwei Detektoren (8, 9) ausgeführt wird, wobei die beiden Detektoren einander gegenüberliegend durch eine Grundsäule (1, 6, 7) gehalten sind, die um eine Rotationsachse schwenkbar ist, dadurch gekennzeichnet, daß

- man jeden der beiden Detektoren mit einer Schwenkachse in Visierrichtung versieht, die beiden Schwenkachsen parallel zur Rotationsachse der Grundsäule sind und wobei die Rotationsachse der Grundsäule in einer Ebene liegt, die durch die beiden Schwenkachsen bestimmt ist,

- man durch Verschwenken die Visierrichtung eines jeden Detektors ausrichtet, um so ein Visierzentrum (262) zu bestimmen, das einen Abstand E zu der Rotationsachse aufweist.

2. Verfahren nach Anspruch 1, dadurch gekennzeichnet, daß die Untersuchung eine Tomographie ist, daß man den Körper untersucht, indem man die Grundsäule um die Rotationsachse dreht, und daß das Visierzentrum sich außerhalb der Rotationsachse in einer Ebene quer zu dieser Achse befindet.

3. Verfahren nach Anspruch 1 oder Anspruch 2, dadurch gekennzeichnet, daß
   - man die beiden Detektoren zusammen um einen Winkel von unter 180° verdreht.

4. Verfahren nach einem der Ansprüche 1 bis 3, dadurch gekennzeichnet, daß
   - man das Rotationszentrum und das Visierzentrum während der Rotation verschiebt, und daß
   - man den Wert des Abstandes während der Rotation beibehält.

5. Verfahren nach Anspruch 4, dadurch gekennzeichnet, daß man das Rotationszentrum horizontal verschiebt, während man das Visierzentrum vertikal verschiebt.

6. Verfahren nach einem der Ansprüche 1 bis 5, dadurch gekennzeichnet, daß
   - man die Visierrichtungen der beiden Detektoren derart ausrichtet, daß ihre Hauptvisierrichtungen untereinander einen Winkel von 90° bilden.

7. Verfahren nach einem der Ansprüche 1 bis 6, dadurch gekennzeichnet, daß
   - man die Visierrichtungen beider Detektoren um einen gleichen Winkel in bezug auf die Ausrichtungen der Basis desorientiert, die sie haben, wenn die Visierrichtungen parallel sind.

8. Verfahren nach einem der Ansprüche 1 bis 7, dadurch gekennzeichnet, daß
   - man die beiden Detektoren um einen gleichen Winkel in bezug auf die Ausrichtungen der Basis verschwenkt, die sie haben, wenn sie parallel sind.

9. Verfahren nach einem der Ansprüche 1 bis 8, dadurch gekennzeichnet, daß
   - man jeden der beiden Detektoren um einen Winkel $\Theta_1$ bzw. $\Theta_2$ verschwenkt, derart, daß die Summe der beiden Winkel gleich ist dem Aus-

druck $(\pi - \beta)/2$, wobei $\beta$ ein Untersuchungs-Komplementärsektor über einen Hauptsektor von 180° hinaus ist.

10. Verfahren nach einem der Ansprüche 1 bis 9, dadurch gekennzeichnet, daß
    - man die Visierrichtungen um vordefinierte Winkel desorientiert.

11. Verfahren nach einem der Ansprüche 1 bis 10, dadurch gekennzeichnet, daß
    - man die Visierrichtungen gleichzeitig durch Verschwenken der Detektoren desorientiert und ihre Detektorenfläche mit einer geneigten Visiervorrichtung versieht.

12. Verfahren nach einem der Ansprüche 1 bis 11, dadurch gekennzeichnet, daß
    - man die Untersuchung beginnt, indem man die Liege genau symmetrisch unter die beiden Detektoren anordnet.

13. Verfahren nach einem der Ansprüche 1 bis 12, dadurch gekennzeichnet, daß
    - man den Patienten hebt, ohne die Grundsäule seitlich zu verschieben.

14. Verfahren nach einem der Ansprüche 1 bis 12, dadurch gekennzeichnet, daß
    - man die Grundsäule in einer Kreisbewegung um den Patienten verschiebt, ohne die Liege zu verschieben.

## Claims

1. Acquisition method for tomographic images (36) in the course of an examination in nuclear medicine, carried out with a gamma camera with two detector heads (8, 9), these two heads being held (1, 6, 7) opposite each other by a base turning about a rotation axis, characterised by the fact that
   - each of these two heads is provided with an angulation axis for its view direction, these two angulation axes being parallel to the rotation axis of the base, this rotation axis of the base being contained within the plane defined by the said two angulation axes;
   - the view direction of each of the heads is orientated (24, 25) from the point of view of angulation, thus defining a view centre (262) situated at a distance (E) from the said rotation axis.

2. Method according to claim 1, characterised by the fact that the examination is a tomographic examination, inasmuch as the body is explored by turning the base about a rotation axis and the view centre is situated outside the rotation axis, in a plane transversal to the said axis.

3. Method according to claim 1 or claim 2, characterised by the fact that
    - the two heads together are caused to rotate through an angle well below 180°.

4. Method according to any one of claims 1 to 3, characterised by the fact that
    - the position of the rotation centre and that of the view centre are adjusted in the course of the rotation, and that
    - the magnitude of the distance is maintained throughout the rotation.

5. Method according to claim 4, characterised by the fact that the rotation centre is displaced horizontally while the view centre is displaced vertically.

6. Method according to any one of claims 1 to 5, characterised by the fact that
    - the view directions of the two heads are orientated in such a manner that their main view directions form between them an angle of 90°.

7. Method according to any one of claims 1 to 6, characterised by the fact that
    - the view directions of the two heads are readjusted so that they form an equal angle in relation to basic orientations which they have when these view directions are parallel.

8. Method according to any one of claims 1 to 7, characterised by the fact that
    - the two heads are angulated at an equal angle in relation to basic orientations which they have when they are parallel.

9. Method according to any one of claims 1 to 8, characterised by the fact that
    - each of the two heads is angulated at an angle of $\theta_1$ and $\theta_2$ respectively such that the sum of these two angles is equal to $(\pi - \beta)/2$ where $\beta$ is a complementary exploration sector beyond a main sector of 180°.

10. Method according to any one of claims 1 to 9, characterised by the fact that
    - the view directions are re-adjusted at preselected angles.

11. Method according to any one of claims 1 to 10, characterised by the fact that
    - the view directions are re-adjusted both by angulating the detector heads and by providing their detection surface with an inclined collimator.

12. Method according to any one of claims 1 to 11, characterised by the fact that
    - the examination is commenced by placing the couch in a position substantially symmetrical underneath the two detector heads.

13. Method according to any one of claims 1 to 12, characterised by the fact that
    - the patient is raised without displacing the base laterally.

14. Method according to any one of claims 1 to 12, characterised by the fact that
    - the position of the base is adjusted by a circular movement around the patient without displacing the couch.

FIG_1

FIG_2a  ART ANTERIEUR

FIG_2b

FIG_3a

FIG_4a

FIG_3b

FIG_4b

FIG_3c

FIG_4c

FIG_5

FIG_6

FIG_7b

FIG_7a